(19) Europäisches Patentamt
European Patent Office
Office européen des brevets

(11) Numéro de publication: 0 093 643
A1

(12) # DEMANDE DE BREVET EUROPEEN

(21) Numéro de dépôt: 83400801.3

(22) Date de dépôt: 22.04.83

(51) Int. Cl.[3]: C 07 D 453/02
A 61 K 31/435

(30) Priorité: 04.05.82 FR 8207703

(43) Date de publication de la demande: 09.11.83 Bulletin 83/45

(84) Etats contractants désignés: BE CH DE FR GB IT LI LU NL SE

(71) Demandeur: PHARMUKA LABORATOIRES
35, Quai du Moulin de Cage
F-92231 Gennevilliers(FR)

(72) Inventeur: Renault, Christian Louis Albert
10 Allée Réaumur
F-95150 Taverny(FR)

(72) Inventeur: Le Fur, Gérard Roger
35, rue du Progrès
F-92350 Plessis Robinson(FR)

(74) Mandataire: Leboulenger, Jean et al,
P C U K PRODUITS CHIMIQUES UGINE KUHLMANN
Service Propriéte Industrielle Tour Manhattan
F-92087 Paris La Défense 2 Cedex 21(FR)

(54) **Isomères dextrogyres de dérivés de l'aza-1 bicyclo (2,2,2)octane, leur procédé de préparation et médicaments les contenant.**

(57) Composés, utiles comme médicaments, de formule:

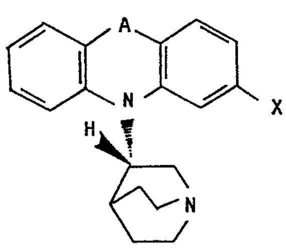

($I_a$)

dans laquelle A est un atome de soufre ou un groupe $-CH_2-CH_2-$, X est un atome d'hydrogène ou un groupe $-SO_2N(CH_3)_2$ et la configuration absolue de l'atome de carbone en position 3 du cycle aza-1 bicylo(2,2,2)octane est sinister (S).

EP 0 093 643 A1

0093643



Isomères dextrogyres de dérivés de l'aza-1 bicyclo[2,2,2]octane, leur procédé de préparation et médicaments les contenant

La présente invention a pour objet les isomères dextrogyres correspondant aux composés racémiques de formule :

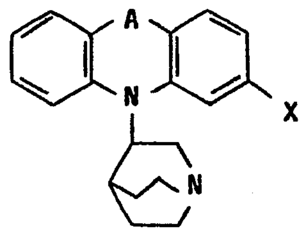

(I)

dans laquelle A est un atome de soufre ou un groupe $-CH_2-CH_2-$ et X est un atome d'hydrogène ou un groupe $-SO_2N(CH_3)_2$.

L'invention a également pour objet un procédé de préparation de ces isomères dextrogyres et leur utilisation comme médicaments.

Les composés racémiques de formule (I) sont des composés connus, doués de propriétés pharmacologiques intéressantes, qui sont utilisables en particulier pour le traitement des ulcères gastriques et duodénaux et comme antidépresseurs (cf. les brevets français 2 318 638 et 2 052 991).

La formule (I) comportant un atome de carbone asymétrique (carbone en position 3 du cycle aza-1 bicyclo[2,2,2]octane), les composés racémiques répondant à cette formule sont dédoublables en deux inverses optiques ou énantiomères.

Les composés selon la présente invention, qui sont les énantiomères dextrogyres, répondent à la formule :

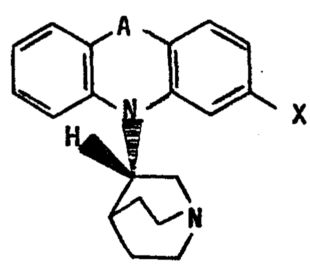

$(I_a)$

dans laquelle A et X ont la même signification que dans la formule (I) et dans laquelle la configuration absolue de l'atome de carbone en position 3 du cycle aza-1 bicyclo(2,2,2) octane est sinister (S).

Les composés de formule ($I_a$) peuvent être préparés par condensation du phénylsulfonyloxy-3 (R) [aza-1 bicyclo(2,2,2)octane] avec les composés de formule :

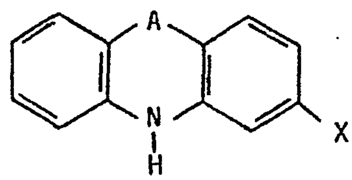

(II)

dans laquelle A et X ont la même signification que dans la formule (I), selon un procédé analogue à celui décrit dans les brevets français précédemment cités pour la préparation des composés de formule (I) à partir du phénylsulfonyloxy-3 [aza-1 bicyclo(2,2,2)octane] racémique et des composés de formule (II). Une telle condensation peut être effectuée par exemple au sein d'un solvant inerte tel qu'un hydrocarbure aromatique (toluène, xylène, etc...) ou un mélange d'un hydrocarbure aromatique et d'un solvant aprotique polaire (par exemple l'hexaméthylphosphorotriamide), en présence d'hydrure de sodium, à une température de 80°C à 110°C.

Les composés ($I_a$) obtenus à l'état brut par le procédé décrit précédemment peuvent être purifiés par des méthodes classiques, physiques (cristallisation, chromatographie) ou chimiques (formation de sels et régénération de la base par traitement des sels en milieu alcalin).

Les composés ($I_a$) sous forme de bases libres peuvent être transformés en sels d'addition avec un acide minéral ou organique par action d'un tel acide au sein d'un solvant approprié.

Les énantiomères dextrogyres de formule ($I_a$) présentent, dans les tests pharmacologiques en rapport avec les utilisations thérapeutiques des racémiques de formule (I), une activité supérieure à celle de ces derniers. Ils présentent donc un avantage certain par rapport aux racémiques correspondants puisqu'ils permettent d'obtenir

un effet thérapeutique équivalent avec une posologie plus faible. Un tel résultat était inattendu.

Les exemples suivants illustrent l'invention sans la limiter.

EXEMPLE 1 : N,N-diméthyl [aza-1 bicyclo(2,2,2)octyl-3(S)]- 10 10 H-phénothiazinesulfonamide-2

On place sous atmosphère d'azote 15,3 g de N,N-diméthyl phénothiazinesulfonamide-2 et 160 ml de xylène anhydre. On ajoute 1,5 g d'hydrure de sodium à 80 % dans l'huile et 50 ml d'hexaméthylphosphorotriamide. On porte le mélange à 80°C et ajoute une solution de 9,2 g de phénylsulfonyloxy-3 (R) [aza-1 bicyclo(2,2,2)octane] dans 60 ml de xylène anhydre. Après 20 heures d'agitation, on refroidit, ajoute 50 ml d'eau, décante et extrait la phase aqueuse avec 50 ml d'acétate d'éthyle. On extrait la phase organique (phase acétate d'éthyle) avec 3 fois 50 ml d'une solution aqueuse N d'acide méthanesulfonique et 2 fois 50 ml d'eau. On lave les phases aqueuses rassemblées avec 100 et 50 ml d'éther, puis alcalinise le milieu par addition de 12,5 ml d'une solution concentrée d'ammoniaque et extrait l'huile qui relargue avec 3 fois 100 ml d'acétate d'éthyle. On lave la phase organique avec 50 ml d'eau, la sèche sur du sulfate de magnésium et l'évapore à sec sous pression réduite. On obtient 6,8 g d'un produit que l'on cristallise dans 50 ml d'acétone. On recueille ainsi 4,9 g de N,N-diméthyl [aza-1 bicyclo(2,2,2)octyl-3 (S)] -10 10 H-phénothiazinesulfonamide-2 fondant à 201°C. Après 2 cristallisations dans le méthanol, on récupère 2,6 g de N,N-diméthyl [aza-1 bicyclo(2,2,2) octyl-3 (S)] -10 10H-phénothiazinesulfonamide-2 présentant les caractéristiques suivantes :

point de fusion 201°C

pouvoir rotatoire spécifique (mesuré sur une solution à 1 % du produit dans une solution aqueuse N d'acide méthanesulfonique):

$$\alpha_D^{25} = + 35,7°$$

pureté optique (déterminée par la méthode calorimétrique décrite par C. FOUQUEY et J. JACQUES, Tetrahedron, 23, (1967), 4009) :

$> 97\ \%$

Le phénylsulfonyloxy-3 (R) [aza-1 bicyclo(2,2,2)octane] peut être préparé par action du chlorure de benzènesulfonyle sur l'[aza-1 bicyclo(2,2,2)octan]-ol-3 (R) au sein d'un solvant chloré selon une méthode identique à celle utilisée par E.E. MIKHLINA et Coll. (J. Gen. Chem. USSR, 30, (1960), 2953-8) pour la préparation du phénylsulfonyloxy-3 [aza-1 bicyclo(2,2,2)octane] racémique à partir de l'[aza-1 bicyclo(2,2,2)octan]-ol-3 racémique. Le phénylsulfonyloxy-3 (R) [aza-1 bicyclo(2,2,2)octane] présente les caractéristiques suivantes :

point de fusion $< 50°C$

pouvoir rotatoire spécifique (mesuré sur une solution à 3 % du produit dans une solution aqueuse N d'acide chlorhydrique) :

$$\alpha_D^{23} = -16,4°$$

L'[aza-1 bicyclo(2,2,2)octan]-ol-3 (R) peut être préparé comme indiqué par B. RINGDAHL et Coll., Acta Pharm. Sued., 16, (1979), 281.

EXEMPLE 2 : [aza-1 bicyclo(2,2,2)octyl-3 (S)]-5 dihydro-10,11 5H-dibenz (b,f) azépine

On place sous atmosphère d'azote 4,5 g de phénylsulfonyloxy-3 (R) [aza-1 bicylo(2,2,2)octane], 0,75 g d'hydrure de sodium à 80 % dans l'huile et 40 ml de toluène anhydre. On porte le mélange à 105-110°C et ajoute, en 2 heures, une solution de 4,2 g de dihydro-10,11 5H-dibenz (b,f) azépine et de 3 ml de N-méthylpyrrolidone-2 dans 15 ml de toluène anhydre. On maintient à 105-110°C pendant encore 30 minutes, refroidit, ajoute 2 ml d'éthanol et 20 ml d'eau. On décante et extrait la phase aqueuse avec 20 ml de toluène. On extrait la phase organique avec 2 fois 20 ml d'une solution aqueuse normale d'acide méthanesulfonique puis avec 3 fois 10 ml d'eau. On alcalinise

les phases aqueuses rassemblées par addition de 3,5 ml d'une solution concentrée d'ammoniaque et extrait l'huile qui relargue avec 5 fois 50 ml de toluène. On sèche la phase organique sur du sulfate de magnésium et l'évapore à sec sous pression réduite. Le résidu obtenu (3,1 g) est fixé sur une colonne de gel de silice et on élue ensuite avec un mélange de 97 parties en volume de chloroforme et 3 parties en volume de diéthylamine. On récupère ainsi 2,6 g du produit recherché à l'état brut, que l'on cristallise dans 3 ml d'acétonitrile. On obtient alors 2,2 g d'[aza-1 bicyclo(2,2,2)octyl-3 (S)]-5 dihydro-10,11 5H-dibenz (b,f) azépine qui présente les caractéristiques suivantes :

point de fusion : 144-145°C

pouvoir rotatoire spécifique (mesuré sur une solution à 3 % du produit dans une solution aqueuse N d'acide méthanesulfonique) :

$$\alpha_D^{23} = + 86,8°$$

pureté optique (déterminée par la méthode précédemment indiquée) : > 95 %.

En recristallisant trois fois le produit précédent dans l'acétonitrile, on obtient 0,6 g d'[aza-1 bicyclo(2,2,2)octyl-3 (S)]-5 dihydro-10,11 5H-dibenz (b,f) azépine pure, qui présente un point de fusion de 146-147°C et une pureté optique, déterminée par la méthode précédemment indiquée, de 99 %, et dont le pouvoir rotatoire spécifique (mesuré sur une solution à 1 % du produit dans l'acide méthanesulfonique N) est $\alpha_D^{25} = + 88°$

PROPRIETES PHARMACOLOGIQUES

1°) Activité antisécrétoire :

L'activité inhibitrice de la sécrétion gastrique du composé de l'exemple 1 a été comparée à celle du racémique correspondant à l'aide du test d'hypersécrétion gastrique stimulée par la pentagastrine chez le chien porteur d'une poche de Heidenhein.

Trois chiens "mongrels", porteurs d'une poche de Heidenhein, à jeun depuis 18 heures, reçoivent pendant 4 heures, par perfusion veineuse, 4 mg/kg/heure de pentagastrine sous un volume de 30 ml/heure. La sécrétion de la poche est recueillie dans un flacon qui est changé toutes les 15 minutes. Une heure et demie après le début de la perfusion, le produit à étudier est administré par voie orale dans une gélule n° 000. La dose efficace 50 % (DE 50) est la dose de produit qui diminue de 50 % le débit acide horaire enregistré pendant la 3ème ou 4ème heure de l'expérimentation par rapport au débit acide horaire enregistré lors de la deuxième heure de la perfusion.

Les résultats obtenus sont rassemblés dans le tableau ci-après, où les doses sont exprimées en produit sous forme de base :

| Produit | $DE_{50}$ en mg/kg (voie orale) |
|---|---|
| Exemple 1 | 0,7 |
| racémique correspondant | 1 |

L'activité inhibitrice de la sécrétion gastrique du composé de l'exemple 1 est donc supérieure à celle du racémique correspondant.

2°) Activité antidépressive :

L'activité antidépressive de la quinupramine ayant été reliée au blocage des récepteurs muscariniques centraux [cf. G. LE FUR, l'Encéphale, 6, 303, (1980)], l'activité antidépressive du composé de l'exemple 2 a été comparée à celle du racémique correspondant (quinupramine) en mesurant les affinités respectives de ces produits pour les récepteurs muscariniques de l'acétylcholine. Ces affinités sont déterminées en mesurant l'aptitude des produits à déplacer le benzilate de quinuclidinol-3 tritié ($^3$H-QNB) de son site de liaison

et sont exprimées par une valeur $IC_{50}$ qui est la concentration de produit (en nanomoles par litre) nécessaire pour obtenir une inhibition de 50 % de la liaison du $^{3}H$-QNB. Les mesures ont été effectuées selon le protocole de H.I. YAMAMURA et Coll., Proc. Nat. Acad. Sci (USA), 71, 1725, (1974), en utilisant des membranes de striatum de cerveau de rat.

Les résultats obtenus sont rassemblés dans le tableau ci-dessous :

| Produit | $IC_{50}$ (en nM/l) |
|---|---|
| Exemple 2 | 2,8 |
| Quinupramine | 5,6 |

Le composé de l'exemple 2 est donc deux fois plus actif que le racémique correspondant.

PROPRIETES TOXICOLOGIQUES

La toxicité aiguë des composés de formule ($I_a$) est sensiblement identique à celle des racémiques correspondants.

UTILISATION THERAPEUTIQUE

Les composés de formule ($I_a$) et leurs sels avec un acide pharmaceutiquement acceptable peuvent être utilisés en thérapeutique humaine comme matière active de médicament, en particulier de médicament antidépresseur et de médicament antisécrétoire pour le traitement des ulcères gastriques et duodénaux.

Un tel médicament contient, outre la matière active, un

véhicule pharmaceutiquement acceptable tel que ceux communément utilisés dans le domaine pharmaceutique et peut se présenter sous forme de comprimés, capsules, gélules, suppositoires, solution ingérable ou injectable, etc...

La posologie dépend des effets recherchés et de la voie d'administration utilisée. Par exemple, par voie orale, elle se situe en général pour un adulte entre 10 et 200 mg par jour de substance active.

**Revendications de brevet**

1. Composés de formule :

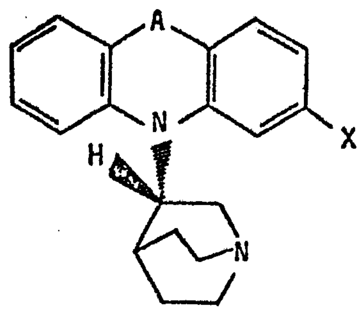

$(I_a)$

dans laquelle A est un atome de soufre ou un groupe $-CH_2-CH_2-$, X est un atome d'hydrogène ou un groupe $-SO_2N(CH_3)_2$ et la configuration absolue de l'atome de carbone en position 3 du cycle aza-1 bicyclo (2,2,2)octane est sinister (S).

2. Composé selon la revendication 1, caractérisé en ce que A est un atome de soufre et X est un groupe $-SO_2N(CH_3)_2$.

3. Composé selon la revendication 1, caractérisé en ce que A est un groupe $-CH_2-CH_2-$ et X est un atome d'hydrogène.

4. Procédé de préparation des composés selon la revendication 1, caractérisé en ce que l'on condense le phénylsulfonyloxy-3 (R) [aza-1 bicyclo(2,2,2)octane] avec un composé de formule :

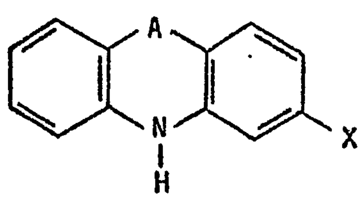

(II)

dans laquelle A et X ont la même signification que dans la formule $(I_a)$

de la revendication 1, au sein d'un solvant inerte, en présence d'hydrure de sodium.

5. Médicament, particulièrement utile comme médicament antidépresseur ou comme médicament antisécrétoire pour le traitement des ulcères gastriques et duodénaux, contenant une substance active et un véhicule pharmaceutiquement acceptable, caractérisé en ce que la substance active est un composé selon la revendication 1 ou un sel d'un tel composé avec un acide pharmaceutiquement acceptable.

6. Médicament, particulièrement utile comme médicament antidépresseur, contenant une substance active et un véhicule pharmaceutiquement acceptable, caractérisé en ce que la substance active est le composé selon la revendication 1 pour lequel A est un groupe $-CH_2-CH_2-$ et X est un atome d'hydrogène ou un sel de ce composé avec un acide pharmaceutiquement acceptable.

7. Médicament, particulièrement utile comme médicament antisécrétoire pour le traitement des ulcères gastriques et duodénaux, contenant une substance active et un véhicule pharmaceutiquement acceptable, caractérisé en ce que la substance active est le composé selon la revendication 1 pour lequel A est un atome de soufre et X est un groupe $-SO_2N(CH_3)_2$ ou un sel de ce composé avec un acide pharmaceutiquement acceptable.

0093643

Office européen des brevets

# RAPPORT DE RECHERCHE EUROPEENNE

Numéro de la demande

EP 83 40 0801

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| X,D | FR-A-2 318 638 (SOGERAS) * Revendications; exemple 1 * | 1,2,4-7 | C 07 D 453/02 A 61 K 31/435 |
| X,D | FR-A-2 052 991 (SOGERAS) * Revendications; exemple 3 * | 1,3-7 | |
| X,D | FR-A-2 034 605 (SOGERAS) * Revendications; exemple 4 * | 1,4-7 | |
| D,A | CHEMICAL ABSTRACTS, vol. 92, 1980, pages 763,764, no. 41726j, Columbus, Ohio, USA B. RINGDAHL et al.: "Facile preparation of the enantiomers of 3-acetoxyquinuclidine and 3-quinuclidinol" & ACTA PHARM. SUEC. 1979, 16(4), 281-283 * Abrégé * | | DOMAINES TECHNIQUES RECHERCHES (Int. Cl. 3) |
| D,A | CHEMICAL ABSTRACTS, vol. 55, 1961, colonne 18727c, Columbus, Ohio, USA E.E. MIKHLINA et al.: "New paths of synthesis of 3-quinuclidineacetic acid" & ZHUR. OBSHCHEI KHIM. 30, 2970-2977, 1960 * Abrégé * | | C 07 D 453/00 A 61 K 31/00 |

Le présent rapport de recherche a été établi pour toutes les revendications

| Lieu de la recherche | Date d'achèvement de la recherche | Examinateur |
|---|---|---|
| LA HAYE | 03-08-1983 | NUYTS A.M.K.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant